# EUROPEAN PATENT APPLICATION

(11) **EP 3 780 118 A1**
(43) Date of publication of application: **17.02.2021**
(21) Application number: 19780760.5
(22) Date of filing: 15.03.2019
(51) Int. Cl.: H01L 29/786, C07D 495/04, C07D 495/14, H01L 51/05, H01L 51/30

(54) **ORGANIC SEMICONDUCTOR ELEMENT, COMPOSITION, METHOD OF PURIFYING COMPOUND, AND APPLICATION THEREOF**

(30) Priority: 03.04.2018 JP 2018071688
(71) Applicant: FUJIFILM CORPORATION, Minato-ku Tokyo 106-8620 (JP)
(72) Inventor: WATANABE, Tetsuya, Ashigarakami-gun, Kanagawa 258-8577 (JP); TANI, Yukio, Ashigarakami-gun, Kanagawa 258-8577 (JP)
(74) Representative: Hoffmann Eitle
(86) International application number: PCT/JP2019/010860
(87) International publication number: WO 2019/193953

(57) **Abstract**

Provided are an organic semiconductor element, a composition, an organic semiconductor composition, an organic semiconductor film, a method of producing a composition, a method of manufacturing an organic semiconductor element, and a method of purifying a compound. The organic semiconductor element includes an organic semiconductor film formed by forming a composition into a film, in which the composition contains a compound represented by the following formula (where R¹ to R⁸ each independently represent a hydrogen atom or a substituent), and a total content of sodium element, potassium element, silicon element, and aluminum element in the composition is 50 ppm or less. The organic semiconductor element has high heat resistance of carrier mobility.

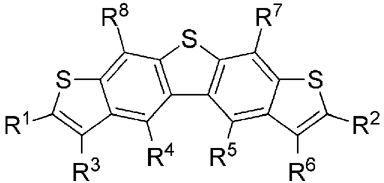

## Description

### BACKGROUND OF THE INVENTION

### 1. Field of the Invention

The present invention relates to an organic semiconductor element, a composition, an organic semiconductor composition, an organic semiconductor film, a method of producing a composition, a method of manufacturing an organic semiconductor element, and a method of purifying a compound.

### 2. Description of the Related Art

Organic semiconductor elements using organic semiconductor materials are expected to be superior in various aspects to elements using inorganic semiconductor materials such as silicon in the related art, and are thus drawing great attention. Examples of the organic semiconductor elements using organic semiconductor materials include a photoelectric conversion element such as an organic thin film solar cell or a solid-state imaging element using an organic semiconductor material as a photoelectric conversion material, and an organic transistor (referred to as an organic thin film transistor in some cases) having non-light-emitting properties. The organic semiconductor elements using organic semiconductor materials are likely to make it possible to prepare large area elements at a lower temperature and lower costs compared to the elements using inorganic semiconductor materials. Further, since the characteristics of the materials can be easily changed by changing the molecular structure, the materials show a wide variation and can realize functions or elements that cannot be obtained by inorganic semiconductor materials.

Regarding materials for an organic transistor which is an organic semiconductor element, the use of compounds having a fused ring in a semiconductor active layer is examined so as to improve carrier mobility and improve transistor performance.

Here, as materials for an organic transistor, compounds having a thieno[3,2-f:4,5-f]bis[1]benzothiophene (hereinafter, also referred to as TBBT) structure are known. For example, Tetrahedron 66 (2010) 8778-8784 discloses a method of synthesizing a compound C6-TBBT obtained by substituting TBBT with an alkyl group having 6 carbon atoms or a compound C12-TBBT obtained by substituting TBBT with an alkyl group having 12 carbon atoms and discloses absorption/emission spectra and cyclic voltammetry (CV) as physical properties of the compound.

In addition, JP2015-195361A describes a coating solution for a non-light emitting organic semiconductor device including a compound having a TBBT structure and a solvent having a boiling point of 100°C or higher.

### SUMMARY OF THE INVENTION

In recent years, from the viewpoint of improving the performance of an organic thin film transistor, there has been a demand for further improvement in carrier mobility of the organic thin film transistor. Particularly, it is required that after a photoresist used for patterning an organic semiconductor film is heated under heating conditions, the carrier mobility can be kept high, that is, the heat resistance of carrier mobility is provided.

The present inventors have found that in a case where an organic semiconductor element is produced by preparing a compound having a TBBT structure according to JP2015-195361A and Tetrahedron 66 (2010) 8778-8784, and forming the compound into a film to form an organic semiconductor film, further improvement is required from the viewpoint of heat resistance of carrier mobility.

An object of the present invention is to provide an organic semiconductor element having high heat resistance of carrier mobility.

As a result of intensive investigations to achieve the above object, the present inventors have found that a film obtained by purifying a compound having a TBBT structure and using a composition having a low specific ion content has high heat resistance of carrier mobility in a case where the film is formed into an organic semiconductor element, and accomplished the present invention.

The present invention as specific means for achieving the above object has the following constitution.
[1] An organic semiconductor element comprising: an organic semiconductor film formed by forming a composition into a film,
   in which the composition contains a compound represented by Formula 1, and
   a total content of sodium element, potassium element, silicon element, and aluminum element in the composition is 50 ppm (where ppm refers to parts per million) or less, 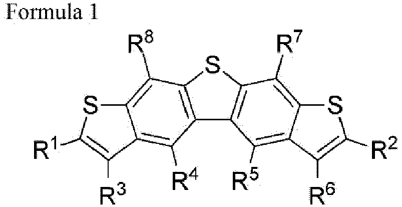 in Formula 1, R¹ to R⁸ each independently represent a hydrogen atom or a substituent.
[2] The organic semiconductor element according to [1], in which the compound represented by Formula 1 is a compound represented by Formula 2, 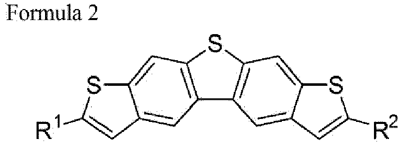 in Formula 2, R¹ and R² each independently represent an alkyl group which may have a substituent or an aromatic group which may have a substituent.
[3] The organic semiconductor element according to [1] or [2], in which a content of the silicon element in the composition is 30 ppm or less.
[4] The organic semiconductor element according to any one of [1] to [3], in which the organic semiconductor element is an organic transistor.
[5] A composition comprising: a compound represented by Formula 1,
   in which a total content of sodium element, potassium element, silicon element, and aluminum element in the composition is 50 ppm or less, 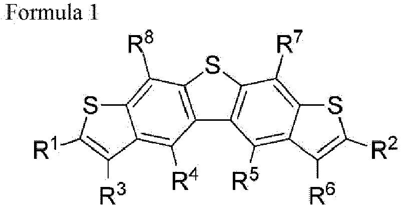 in Formula 1, R¹ to R⁸ each independently represent a hydrogen atom or a substituent.
[6] The composition according to [5], in which a content of the silicon element in the composition is 30 ppm or less.
[7] An organic semiconductor composition comprising: the composition according to [5] or [6]; and a solvent.
[8] An organic semiconductor film formed by forming the organic semiconductor composition according to [7] into a film.
[9] A method of producing a composition comprising: a step of subjecting a compound represented by Formula 1 to decompression purification at a temperature of 150°C or higher or Soxhlet extraction, 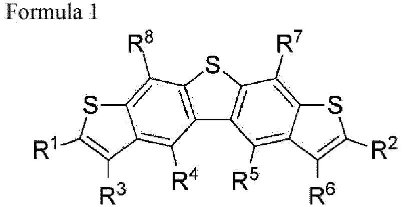 in Formula 1, R¹ to R⁸ each independently represent a hydrogen atom or a substituent.
[10] A method of manufacturing an organic semiconductor element comprising: a step of producing a composition by the method of producing a composition according to [9];
   a step of producing an organic semiconductor composition by mixing the composition and a solvent; and
   a step of forming an organic semiconductor film by forming the organic semiconductor composition into a film.
[11] The method of manufacturing an organic semiconductor element according to [10], in which the step of forming an organic semiconductor film includes a step of applying or printing the organic semiconductor composition onto a substrate, and then drying the organic semiconductor composition to form an organic semiconductor film.
[12] A method of purifying a compound comprising: subjecting a compound represented by Formula 1 to decompression purification at a temperature of 150°C or higher or Soxhlet extraction, 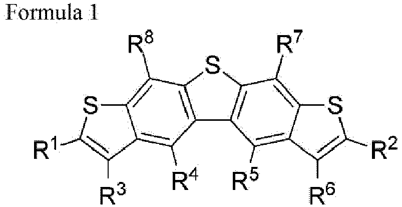 in Formula 1, R¹ to R⁸ each independently represent a hydrogen atom or a substituent.
   [101] A composition obtained by subjecting a compound represented by Formula 1 to decompression purification at a temperature of 150°C or higher or Soxhlet extraction,
      in which a total content of sodium element, potassium element, silicon element, and aluminum element in the composition is 50 ppm or less, 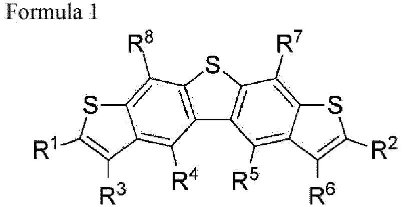 in Formula 1, R¹ to R⁸ each independently represent a hydrogen atom or a substituent.
   [102] An organic semiconductor element comprising: an organic semiconductor film formed by forming a composition, which is obtained by subjecting a compound represented by Formula 1 to decompression purification at a temperature of 150°C or higher or Soxhlet extraction, into a film,
      in which a total content of sodium element, potassium element, silicon element, and aluminum element in the composition is 50 ppm or less, 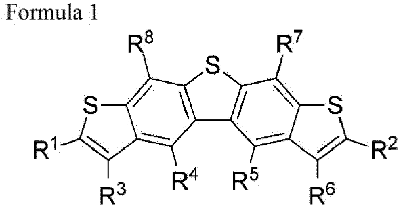 in Formula 1, R¹ to R⁸ each independently represent a hydrogen atom or a substituent.

According to the present invention, it is possible to provide an organic semiconductor element having high heat resistance of carrier mobility.

### BRIEF DESCRIPTION OF THE DRAWINGS

Fig. 1 is a schematic view showing a cross section of an exemplary structure of an organic transistor which is a bottom gate/top contact-type element.
Fig. 2 is a schematic view showing a cross section of an example of an organic transistor which is a bottom gate/bottom contact-type element.

### DESCRIPTION OF THE PREFERRED EMBODIMENTS

Hereinafter, the present invention will be described in detail. The description of the constituent requirements described below may be made based on representative embodiments or specific examples, but the present invention is not limited to such embodiments. In the present specification, a numerical range described using "to" means a range including the numerical values described before and after "to" as a lower limit and an upper limit, respectively.

In the present invention, unless otherwise specified, a hydrogen atom used in the description of each formula represents a hydrogen atom including an isotope (deuterium atom or the like). Further, an atom constituting a substituent represents an atom including an isotope thereof.

### [Organic Semiconductor Element, Composition, Organic Semiconductor Composition, and Organic Semiconductor Film]

An organic semiconductor element according to an embodiment of the present invention is an organic semiconductor element including an organic semiconductor film formed by forming a composition into a film,
in which the composition contains a compound represented by Formula 1, and
a total content of sodium element, potassium element, silicon element, and aluminum element in the composition is 50 ppm or less.

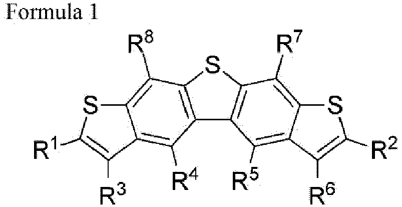

In Formula 1, R¹ to R⁸ each independently represent a hydrogen atom or a substituent.

With these configurations, the organic semiconductor element according to the embodiment of the present invention has high heat resistance of carrier mobility.

Presumably, though the presumption is not restricted by any theory, the presence of the sodium element, the potassium element, the silicon element, and the aluminum element (hereinafter, referred to as specific elements), particularly, ions of these elements in the composition leads to a disorder in the crystal arrangement in a case where an organic semiconductor film is formed. It is considered that the disorder of the crystal arrangement serves as a starting point, and the organic semiconductor film is cracked by heating under the heating conditions for a photoresist used for patterning the organic semiconductor film, and the carrier mobility is lowered.

On the other hand, in a case where the compounds listed in Table 19 of WO2015/133402A and the compounds not satisfying Formula 1, such as pentacene, described in JP2003-347624A are used, even in a case where the total content of the specific elements is reduced, heat resistance of carrier mobility cannot be increased.

On the other hand, in the present invention, by reducing the total content of the specific elements and selecting the compound represented by Formula 1, due to the synergistic effect of both the elements and the compound, a disorder in the crystal arrangement in a case where an organic semiconductor film is formed is reduced and thus heat resistance of carrier mobility can be increased.

Hereinafter, preferable embodiments of the organic semiconductor element, a composition, an organic semiconductor composition, and an organic semiconductor film according to the present invention will be described.

On the other hand, the composition according to the embodiment of the present invention is a composition containing the compound represented by Formula 1, and the total content of sodium element, potassium element, silicon element, and aluminum element in the composition is 50 ppm or less.

The organic semiconductor composition according to the embodiment of the present invention contains the composition according to the embodiment of the present invention and a solvent.

The organic semiconductor film according to the embodiment of the present invention is formed by forming the organic semiconductor composition according to the embodiment of the present invention into a film.

The composition, the organic semiconductor composition, and the organic semiconductor film according to the embodiments of the present invention can be used as an organic semiconductor material, and can also be used for producing the organic semiconductor element according to the embodiment of the present invention. In the present specification, the "organic semiconductor material" is an organic material exhibiting semiconductor characteristics. Similar to a semiconductor consisting of an inorganic material, an organic semiconductor material is classified into a p-type (hole-transporting) organic semiconductor material conducting holes as carriers and an n-type (electron-transporting) organic semiconductor materials conducting electrons as carriers. The compound represented by Formula 1 may be used as any of the p-type organic semiconductor material or the n-type organic semiconductor material, but is more preferably used as the p-type. The ease with which the carriers flow in the organic semiconductor is represented by a carrier mobility µ. The higher the carrier mobility µ, the better.

### <Composition>

The composition according to the embodiment of the present invention is a composition containing the compound represented by Formula 1, and the total content of sodium element, potassium element, silicon element, and aluminum element in the composition is 50 ppm or less.

### (Compound Represented by Formula 1)

In the present invention, the composition contains a compound represented by Formula 1.

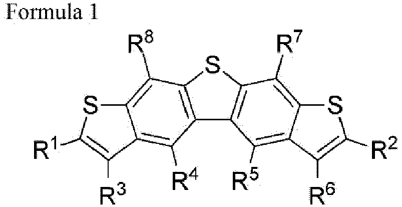

In Formula 1, R¹ to R⁸ each independently represent a hydrogen atom or a substituent.

It is preferable that R¹ and R² each independently represent a substituent. In a case where R¹ and R² each represent a substituent, a preferable substituent is an alkyl group, an alkenyl group, an alkynyl group, an alkoxy group, or an aromatic group, a more preferable substituent is an alkyl group or an aromatic group, and a particularly preferable substituent is an alkyl group.

Although not particularly limited, the alkyl group represented by R¹ and R² is preferably an alkyl group having 1 to 30 carbon atoms, and may be linear, branched, or cyclic. From the viewpoint of increasing heat resistance of carrier mobility, the alkyl group represented by R¹ and R² is preferably a linear alkyl group having 2 to 15 carbon atoms, and is more preferably a linear alkyl group having 2 to 5 carbon atoms and 7 to 10 carbon atoms.

By selecting a specific alkyl chain length or shape, an orbital overlap greatly occurs between molecules, and as a result, carrier mobility can be further improved.

Although not particularly limited, the alkenyl group represented by R¹ and R² is preferably an alkenyl group having 2 to 30 carbon atoms, more preferably an alkenyl group having 3 to 18 carbon atoms, and particularly preferably an alkenyl group having 5 to 13 carbon atoms.

Although not particularly limited, the alkynyl group represented by R¹ and R² is preferably an alkynyl group having 2 to 30 carbon atoms, more preferably an alkynyl group having 3 to 18 carbon atoms, and particularly preferably an alkynyl group having 5 to 13 carbon atoms.

Although not particularly limited, the alkoxy group represented by R¹ and R² is preferably an alkoxy group having 1 to 30 carbon atoms, more preferably an alkoxy group having 3 to 18 carbon atoms, and particularly preferably an alkoxy group having 5 to 13 carbon atoms.

Although not particularly limited, the aromatic group represented by R¹ and R² is preferably an aromatic group having 6 to 30 carbon atoms, more preferably an aromatic group having 6 to 14 carbon atoms, and particularly preferably an aromatic group having 6 carbon atoms.

In a case where the substituent represented by R¹ and R² further has a substituent, a preferable range of the substituent is the same as the range described in [0018] of JP2015-195361A. For example, it is preferable that the substituent has an alkyl group or an alkoxy group as a further substituent of the substituent.

It is preferable that R³ to R⁸ each independently represent a hydrogen atom or a halogen atom. The halogen atom represented by R³ to R⁸ is preferably a fluorine atom. The number of halogen atoms in R³ to R⁸ is preferably 0 to 6, more preferably 0 to 4, particularly preferably 0 to 2, and particularly preferably 0.

In the present invention, the compound represented by Formula 1 is preferably a compound represented by Formula 2.

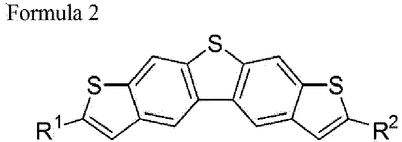

In Formula 2, R¹ and R² each independently represent an alkyl group which may have a substituent or an aromatic group which may have a substituent, and the preferable range is the same as the range in Formula 1.

The total number of carbon atoms of R¹ and R² is each independently preferably 3 to 30, more preferably 7 to 30, particularly preferably 7 to 20, more particularly preferably 7 to 15, even more particularly preferably 7 to 11, and still even more particularly preferably 9 to 11. In a case where the total number of carbon atoms of R¹ and R² is each independently at least the lower limit of the above range, carrier mobility is increased. In a case where the total number of carbon atoms of R¹ and R² is at most the upper limit of the above range, solubility in an organic solvent becomes high.

Specific examples of the compound represented by Formula 1, particularly, a combination of R¹ and R² or a combination of R¹ to R⁸ are shown below. The compound represented by Formula 1 used in the present invention should not be interpreted in a limited manner based on these specific examples. Ph in the following Tables 1 to 3 represents a phenyl group. Unless otherwise specified, an alkyl group represents a linear alkyl group.

**[Table 1]**

| No. | R¹ | R² |
|---|---|---|
| 1 | -H | -H |
| 2 | -CH₃ | -CH₃ |
| 3 | -C₂H₅ | -C₂H₅ |
| 4 | -C₃H₇ | -C₃H₇ |
| 5 | -C₄H₉ | -C₄H₉ |
| 6 | -C₅H₁₁ | -C₅H₁₁ |
| 7 | -C₆H₁₃ | -C₆H₁₃ |
| 8 | -C₇H₁₅ | -C₇H₁₅ |
| 9 | -C₈H₁₇ | -C₈H₁₇ |
| 10 | -C₉H₁₉ | C₉H₁₉ |
| 11 | -C₁₀H₂₁ | -C₁₀H₂₁ |
| 12 | -C₁₁H₂₃ | -C₁₁H₂₃ |
| 13 | -C₁₂H₂₅ | -C₁₂H₂₅ |
| 14 | -C₂₄H₄₉ | -C₂₄H₄₉ |
| 15 | 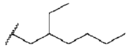 | 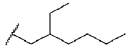 |
| 16 | -OC₆H₁₃ | -OC₆H₁₃ |
| 17 | -SC₆H₁₃ | -SC₆H₁₃ |
| 18 | -C₂H₅OC₄H₉ | -C₂H₅OC₄H₉ |
| 19 | 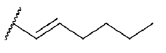 | 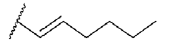 |
| 20 | 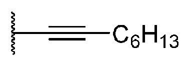 | 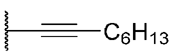 |
| 21 | -C₂H₅C₈F₁₇ | -C₂H₅C₈F₁₇ |

**[Table 2]**

| No. | R¹ | R² |
|---|---|---|
| 22 | -H | 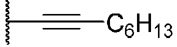 |
| 23 | -Ph | -C₄H₉ |
| 24 | -Ph | -C₆H₁₃ |
| 25 | -Ph | -C₈H₁₇ |
| 26 | -Ph | -C₁₀H₂₁ |
| 27 | -Ph | -C₁₄H₂₉ |
| 28 | -Ph | -C₂₀H₄₁ |
| 29 | -Ph | 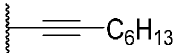 |
| 30 | -C₂H₄Ph | -C₄H₉ |
| 31 | -C₂H₄Ph | -C₆H₁₃ |
| 32 | -C₂H₄Ph | -C₈H₁₇ |
| 33 | -C₂H₄Ph | -C₁₀H₂₁ |
| 34 | -CH₂Ph | -C₁₀H₂₁ |
| 35 | -C₃H₆Ph | -C₁₀H₂₁ |
| 36 | -H | -C₁₀H₂₁ |
| 37 | -Br | -C₂₀H₄₁ |
| 38 | -I | -C₁₈H₃₇ |
| 39 | 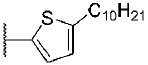 | 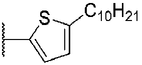 |
| 40 | 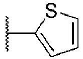 | -C₁₆H₃₂ |
| 41 | 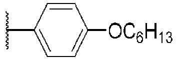 | 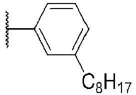 |
| 42 | -CO₂C₈H₁₇ | -CO₂C₈H₁₇ |

**[Table 3]**

| No. | R¹ | R² | R³ | R⁴ | R⁵ | R⁶ | R⁷ | R⁸ |
|---|---|---|---|---|---|---|---|---|
| 101 | -C₆H₁₃ | -C₆H₁₃ | -F | -H | -H | -F | -H | -H |
| 102 | -F | -F | -F | -F | -F | -F | -F | -F |
| 103 | -C₄H₉ | -C₄H₉ | -H | -H | -H | -H | -OC₆H₁₃ | -OC₆H₁₃ |
| 104 | -C₄H₉ | -C₄H₉ | -H | -OC₆H₁₃ | OC₆H₁₃ | -H | -H | -H |
| 105 | -C₄H₉ | -C₄H₉ | 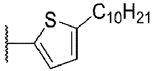 | -H | -H | 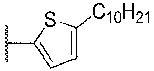 | -H | -H |

The compound represented by Formula 1 preferably has a molecular weight of 3000 or less, more preferably 2000 or less, even more preferably 1000 or less, and particularly preferably 850 or less. In a case where the molecular weight is equal to or less than the above upper limit, solubility in a solvent can be increased, and thus this case is preferable.

On the other hand, from the viewpoint of the film quality stability of the film, the molecular weight is preferably 250 or more, more preferably 300 or more, and even more preferably 350 or more.

The compound represented by Formula 1 can be synthesized with reference to the methods described in JP2015-195361A and Tetrahedron 66 (2010) 8778-8784, or the method described in examples below.

In the synthesis of the compound represented by Formula 1, any reaction condition may be used. As a reaction solvent, any of solvents may be used. Further, in order to promote a ring-forming reaction, an acid or a base is preferably used, and an acid is particularly preferably used. Although optimal reaction conditions vary depending on the structure of the intended compound, the reaction conditions can be set with reference to the specific reaction conditions described in the aforementioned documents or the method described in examples described later.

A synthetic intermediate having various substituents can be synthesized using known reactions in combination. Further, various substituents may be introduced into the intermediate at any stage. After the intermediate is synthesized, it is preferable to purify the intermediate by column chromatography, recrystallization, or the like and then further purify the intermediate by sublimation purification. By sublimation purification, not only can organic impurities be separated, but also inorganic salts, residual solvents, and the like can be effectively removed.

### (Content of Specific Elements)

In the present invention, the total content of sodium element, potassium element, silicon element, and aluminum element in the composition is 50 ppm or less. From the viewpoint of increasing the heat resistance of carrier mobility, the total content of the sodium element, the potassium element, the silicon element, and the aluminum element in the composition is preferably 20 ppm or less, more preferably 10 ppm or less, and particularly preferably 5 ppm or less.

In the present invention, from the viewpoint of further improving heat resistance of carrier mobility, the content of the silicon element in the composition is preferably 30 ppm or less, more preferably 5 ppm or less, and particularly preferably less than 1 ppm.

The specific elements may be present in the form of ion in the composition.

### <Organic Semiconductor Composition>

The organic semiconductor composition according to the embodiment of the present invention contains the composition according to the embodiment of the present invention and a solvent.

### (Solvent)

The solvent used for the organic semiconductor composition will be described.

In a case where the composition according to the embodiment of the present invention is formed into a film on a substrate using a solution process, a film can be formed by a coating method using a solution of an organic semiconductor composition prepared by dissolving or dispersing the composition according to the embodiment of the present invention in a solvent as a coating solution. The solvent may be used alone or plural kinds thereof may be used in combination. The solvent is preferably an organic solvent or water. A preferable aspect of the organic solvent is the same as the preferable aspect of the organic solvent described in [0043] of JP2015-195361A, and this publication is incorporated herein by reference.

### (Concentration of Compound Represented by Formula 1)

The composition according to the embodiment of the present invention used for an organic semiconductor composition contains a compound represented by Formula 1. The concentration of the compound represented by Formula 1 in the organic semiconductor composition is preferably 0.005% to 5% by mass, more preferably 0.01% to 3% by mass, and particularly preferably 0.1% to 2% by mass. By setting the concentration in this range, it is easy to form a film having an arbitrary thickness. Further, it is particularly preferable that the concentration of the compound represented by Formula 1 in the organic semiconductor composition is 0.4% by mass or more since an organic semiconductor film having a large crystal size can be easily formed.

The organic semiconductor composition may include only one kind of the compound represented by Formula 1, or may include two or more kinds thereof.

### (Additive)

The organic semiconductor composition may contain additives such as a surfactant, an antioxidant, a crystallization control agent, a crystal alignment control agent, and a polymer binder. Preferable aspects of the surfactant, the antioxidant, and the polymer binder are the same as the preferable aspects described in [0050], [0051] and [0088] of JP2015-195361A, and this publication is incorporated herein by reference.

### <Application of Organic Semiconductor Element>

The application of the organic semiconductor element is not particularly limited. For example, it is preferable to use the organic semiconductor element for a non-light-emitting organic semiconductor element. In the present specification, the "non-light-emitting organic semiconductor element" means an element which is not intended to emit light. Particularly, the "non-light-emitting organic semiconductor element" means an element which is not intended to emit visible light. In the present specification, the "non-light-emitting" refers to properties by which a light emission efficiency of 1 lm/W or less is obtained in a case where electric currents are applied to an element at a current density of 0.1 mW/cm² at room temperature in the atmosphere. The non-light-emitting organic semiconductor element means an organic semiconductor element excluding a light emitting organic semiconductor element such as an organic electroluminescent element.

The non-light-emitting organic semiconductor element is preferably a non-light-emitting organic semiconductor element using an electronic element having a film layer structure. The non-light-emitting organic semiconductor element includes an organic transistor, an organic photoelectric conversion element (a solid-state imaging element for a photosensor, a solar cell for energy conversion, or the like), a gas sensor, an organic rectifier element, an organic inverter, an information recording element, and the like. The organic photoelectric conversion element can be used for both a photosensor application (solid-state imaging element) and an energy conversion application (solar cell). An organic photoelectric conversion element and an organic transistor are preferable, and an organic transistor is more preferable. That is, the organic semiconductor element according to the embodiment of the present invention is preferably an organic transistor.

It cannot be said that being useful as a material of an organic electroluminescence (EL) element means being useful as a semiconductor material for an organic transistor. This is because the characteristics required for an organic compound vary between an organic EL element and an organic transistor. A mobility of about 10⁻³ cm²/Vs is enough for driving an organic EL element, and for improving organic EL characteristics, it is more important to improve light emission efficiency than to improve electron transporting properties. Therefore, an element having high light emission efficiency and resulting in uniform in-plane light emission is required. Generally, organic compounds having high crystallinity (high mobility) cause light emission defects such as non-uniform in-plane field intensity, non-uniform light emission, and quenching of light emission. Therefore, as materials for an organic EL element, materials having low crystallinity and having high amorphousness (low mobility) are desirable. On the other hand, in a semiconductor material for an organic transistor, extremely high mobility is desired. Accordingly, an organic compound exhibiting highly ordered molecular arrangement and having high crystallinity is required. Further, in order to exhibit high mobility, it is preferable that a π conjugate plane is upright against a substrate.

### <Structure of Organic Semiconductor Element>

A preferable aspect of the structure of the organic semiconductor element according to the embodiment of the present invention will be described by taking a case where the organic semiconductor element according to the embodiment of the present invention is an organic transistor as an example.

The organic transistor preferably includes an organic semiconductor film in a semiconductor active layer.

The organic transistor may further include other layers in addition to the semiconductor active layer.

The organic transistor is preferably used as an organic field effect transistor (FET), and more preferably used as an insulated gate-type FET in which the gate is insulated from channels.

Hereinafter, preferable aspects of the structure of the organic transistor will be described in detail with reference to the drawings, but the present invention is not limited to these aspects.

### (Lamination Structure)

The lamination structure of the organic field effect transistor is not particularly limited and a known structure may be adopted.

As an example of the structure of the organic transistor, a structure (bottom gate/top contact-type) in which an electrode, an insulator layer, a semiconductor active layer (organic semiconductor film), and two electrodes are sequentially arranged on the upper surface of a substrate which is a lowermost layer. In this structure, the electrode on the upper surface of the substrate as the lowermost layer is provided in a portion of the substrate, and the insulator layer is arranged to be in contact with the substrate in a portion other than the electrode. Further, the two electrodes provided on the upper surface of the semiconductor active layer are arranged in a state of being separated from each other.

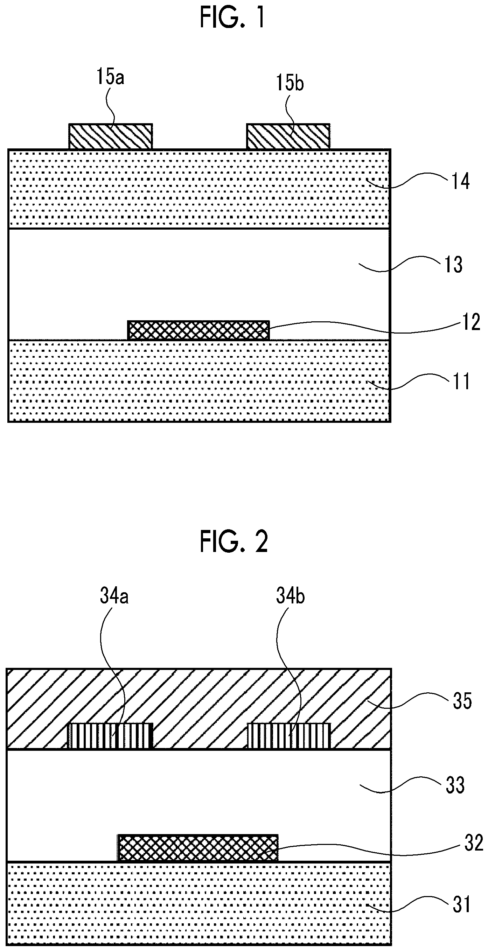

Fig. 1 is a schematic view showing a cross section of an exemplary structure of an organic transistor which is a bottom gate/top contact-type element. In the organic transistor shown in Fig. 1, a substrate 11 is arranged as a lowermost layer, an electrode 12 is provided on a portion of the upper surface thereof, and an insulator layer 13 is provided so as to cover the electrode 12 and to be in contact with the substrate 11 in a portion other than the electrode 12. Further, a semiconductor active layer 14 is provided on the upper surface of the insulator layer 13, and in a portion of the upper surface thereof, two electrodes 15a and 15b are arranged in a state of being separated from each other.

In the organic transistor shown in Fig. 1, the electrode 12 is a gate, and the electrodes 15a and 15b are a drain and a source respectively. The organic transistor shown in Fig. 1 is an insulated gate-type FET in which a channel as a path of electric currents between the drain and the source is insulated from the gate.

As another example of the structure of the organic transistor, a bottom gate/bottom contact-type element can be exemplified.

Fig. 2 is a schematic view showing a cross section of an example of an organic transistor which is a bottom gate/bottom contact-type element. In the organic transistor shown in Fig. 2, a substrate 31 is arranged as a lowermost layer, an electrode 32 is provided in a portion of the upper surface thereof, and an insulator layer 33 is provided so as to cover the electrode 32 and to be in contact with the substrate 31 in a portion other than the electrode 32. Further, a semiconductor active layer 35 is provided on the upper surface of the insulator layer 33, and electrodes 34a and 34b are in a lower portion of the semiconductor active layer 35.

In the organic transistor shown in Fig. 2, the electrode 32 is a gate, and the electrodes 34a and 34b are a drain and a source respectively. The organic transistor shown in Fig. 2 is an insulated gate-type FET in which a channel as a path of electric currents between the drain and the source is insulated from the gate.

As the structure of the organic transistor, a top gate/top contact-type element in which an insulator and a gate electrode are in the upper portion of a semiconductor active layer or a top gate/bottom contact-type element can also be preferably used.

### (Thickness)

In a case where the organic transistor needs to be a thinner transistor, the total thickness of the transistor is preferably, for example, 0.1 to 0.5 µm.

### (Sealing)

In order to enhance the preservability of the organic transistor element by blocking the organic transistor element from the atmosphere or moisture, the entirety of the organic transistor element may be sealed with a metal sealing can, glass, an inorganic material such as silicon nitride, a polymer material such as parylene, a low molecular weight material, or the like.

Hereinafter, preferable aspects of the respective layers of the organic transistor will be described, but the present invention is not limited to the aspects.

### (Substrate)

The organic transistor preferably includes a substrate.

The material of the substrate is not particularly limited, and known materials can be used. Examples of the material include a polyester film such as polyethylene naphthalate (PEN) or polyethylene terephthalate (PET), a cycloolefin polymer film, a polycarbonate film, a triacetylcellulose (TAC) film, a polyimide film, a material obtained by bonding these polymer films to extremely thin glass, ceramics, silicon, quartz, glass, and the like. Among these, silicon is preferable.

### (Electrode)

The organic transistor preferably includes an electrode.

As the material constituting the electrode, known conductive materials such as a metal material such as Cr, Al, Ta, Mo, Nb, Cu, Ag, Au, Pt, Pd, In, Ni, or Nd, an alloy material of these metal materials, a carbon material, and a conductive polymer can be used without particular limitation.

The thickness of the electrode is not particularly limited, and is preferably 10 to 50 nm.

A gate width (or a channel width) W and a gate length (or a channel length) L are not particularly limited. However, a ratio of W/L is preferably 10 or more, and more preferably 20 or more.

### (Acceptor)

The organic transistor preferably includes an acceptor for promoting carrier injection. Preferable examples of the material include known 2,3,5,6-tetrafluoro-7,7,8,8-tetracyanoquinodimethane (F4-TCNQ) and the like.

The thickness of the acceptor is not particularly limited, and is preferably 5 nm or less.

### (Insulator Layer)

The material constituting the insulator layer is not particularly limited as long as the required insulating effect can be obtained. Examples include silicon dioxide, silicon nitride, a fluorine polymer-based insulating material such as polytetrafluoroethylene (PTFE) or CYTOP, a polyester insulating material, a polycarbonate insulating material, an acryl polymer-based insulating material, an epoxy resin-based insulating material, a polyimide insulating material, a polyvinyl phenol resin-based insulating material, and a poly-para-xylylene resin-based insulating material.

A surface treatment may be performed on the upper surface of the insulator layer. For example, an insulator layer in which the silicon dioxide surface thereof is subjected to the surface treatment by application of hexamethyldisilazane (HMDS), octadecyltrichlorosilane (OTS), or β-phenethyltrimethoxysilane can be preferably used, and an insulator layer in which the silicon dioxide surface thereof is subjected to the surface treatment by application of β-phenethyltrimethoxysilane can be more preferably used.

The thickness of the insulator layer is not particularly limited. However, in a case where the film needs to be thinned, the thickness of the insulator layer is preferably 10 to 500 nm, more preferably 20 to 200 nm, and particularly preferably 50 to 200 nm.

### (Semiconductor Active Layer)

In the organic transistor, it is preferable that the semiconductor active layer contains an organic semiconductor film formed by forming the composition according to the embodiment of the present invention into a film.

The semiconductor active layer may be a layer further containing a polymer binder. Further, the semiconductor active layer may contain a residual solvent used at the time of film formation.

The thickness of the semiconductor active layer is not particularly limited. However, in a case where the film needs to be thinned, the thickness is preferably 10 to 400 nm, more preferably 10 to 200 nm, and particularly preferably 10 to 100 nm.

### [Method of Producing Composition and Method of Purifying Compound]

A method of producing a composition according to an embodiment of the present invention includes a step of subjecting a compound represented by Formula 1 to decompression purification at a temperature of 150°C or higher or Soxhlet extraction.

In addition, a method of purifying a compound according to an embodiment of the present invention is to perform decompression purification on the compound represented by Formula 1 at a temperature of 150°C or higher or to perform Soxhlet extraction on the compound.

By these treatments, the content of the specific elements in the composition containing the compound represented by Formula 1 can be reduced, and the composition according to the embodiment of the present invention can be produced.

According to the present invention, compared to the purification particularly using silica gel or silica gel column chromatography, it is possible to reduce the content of the specific elements, and among these, the content of the silicon element (particularly, the content of silicon ions) can be reduced.

In the sublimation purification of the precursor (raw material before synthesis) defined in claim 1 of JP2012-043912A, the range cannot reach the range of the content of the specific elements defined in the present invention.

### <Decompression Purification or Soxhlet Extraction>

In the present invention, the compound represented by Formula 1 is subjected to decompression purification at a temperature of 150°C or higher or Soxhlet extraction.

In the decompression purification or Soxhlet extraction, it is preferable not to use silica gel or a silica gel column particularly.

The decompression purification or the Soxhlet extraction may be performed by only one kind of these treatments, but two or more kinds may be combined.

It is preferable to perform the decompression purification or the Soxhlet extraction in the final step of the purification of the compound represented by Formula 1 from the viewpoint of reducing the content of the specific elements. Other purification methods may be performed as a pretreatment for decompression purification or Soxhlet extraction.

### (Decompression Purification)

The decompression purification is performed at a temperature of at least 150°C or higher, preferably 170°C to 400°C, more preferably 190°C to 350°C, and particularly preferably 200°C to 300°C. It is preferable to perform the purification at a temperature equal to or higher than the above lower limit from the viewpoint of separation from impurities. It is preferable to perform the purification at a temperature equal to or lower than the above upper limit from the viewpoint of suppressing thermal decomposition.

The decompression purification is preferably performed for 1 to 30 hours, more preferably 4 to 20 hours, and particularly preferably 7 to 15 hours.

The decompression purification may be performed while raising the temperature, and it is preferable that the temperature and time in a stage where the temperature is not raised are within the above-mentioned preferable range. The temperature rising rate is not particularly limited and may be, for example, 1 °C/10 minutes to 10 °C/10 minutes.

The decompression purification is preferably performed while flowing an inert gas from the viewpoint of separation from impurities. Examples of the inert gas may include argon gas.

The decompression purification is preferably performed under a pressure condition of 0.01 to 100 mPa, more preferably under a pressure condition of 0.1 to 10 mPa, and particularly preferably under a pressure condition of 1 to 3 mPa.

The state of the compound represented by Formula 1 in the decompression purification is not particularly limited and is preferably a solid state. That is, the decompression purification is preferably sublimation purification from a solid. The state of the compound represented by Formula 1 in the decompression purification can be appropriately selected depending on the compound represented by Formula 1.

### (Soxhlet Extraction)

Soxhlet extraction can be performed using a known Soxhlet extractor.

The solvent used in the Soxhlet extraction is not particularly limited, and an organic solvent can be used. Preferable organic solvents include low polar esters such as ethyl acetate and hydrocarbons such as hexane and toluene. Among these, aromatic hydrocarbons are more preferable.

The solvent may be used alone or two or more kinds thereof may be used. In a case where two or more kinds of solvents are used, it is preferable that hydrocarbons are contained in the solvent in an amount of 90% by mass or more.

### [Method of Manufacturing Organic Semiconductor Element]

A method of manufacturing an organic semiconductor element according to an embodiment of the present invention includes a step of producing a composition by the method of producing a composition according to the embodiment of the present invention,
a step of producing an organic semiconductor composition by mixing the composition and a solvent; and
a step of forming an organic semiconductor film by forming the organic semiconductor composition into a film.

The organic semiconductor element according to the embodiment of the present invention can be easily produced by the production method according to the embodiment of the present invention using film formation by a solution process using an organic semiconductor composition containing a solvent. In the present specification, the film formation by a solution process refers to a method of dissolving an organic compound in a solvent which can dissolve the organic compound and forming a film by using the solution.

However, the organic semiconductor element according to the embodiment of the present invention may be produced by a method other than the above production method. For example, the compound represented by Formula 1 may be formed into a film on the substrate by a vacuum process (a physical vapor deposition method such as a vacuum deposition method, a sputtering method, an ion plating method, or a molecular beam epitaxy method, or a chemical vapor deposition method such as plasma polymerization) without mixing the composition according to the embodiment of the present invention with a solvent.

### <Production of Organic Semiconductor Composition>

In the present invention, the composition (the composition according to the embodiment of the present invention) produced by the method of producing a composition according to the embodiment of the present invention and a solvent are mixed to produce an organic semiconductor composition.

The mixing method is not particularly limited, and the mixing can be performed by a known method.

The organic semiconductor composition after mixing is preferably used for film formation after heating. The temperature of the organic semiconductor composition after mixing is not particularly limited and is preferably 0°C to 200°C, more preferably 15°C to 120°C, and particularly preferably 20°C to 100°C.

### <Film Formation>

In the present invention, the organic semiconductor composition is formed into a film to form an organic semiconductor film. The formed organic semiconductor film can be used for an organic semiconductor element as a semiconductor active layer or the like.

In a method of manufacturing an organic transistor, any method may be used as a method of forming an organic semiconductor film by forming an organic semiconductor composition into a film. It is preferable to form an organic semiconductor film by forming an organic semiconductor composition into a film on a substrate.

During the film formation, the substrate may be heated or cooled. By changing the temperature of the substrate, it is possible to control the film quality or the packing of molecules in the film. The temperature of the substrate is not particularly limited. The temperature is preferably 0°C to 200°C, more preferably 15°C to 120°C, and particularly preferably 20°C to 100°C.

As the step of forming the organic semiconductor film, it is possible to use general methods like a coating method such as a drop casting method, a casting method, a dip coating method, a die coater method, a roll coater method, a bar coater method, or a spin coating method, various printing methods such as an ink jet method, a screen printing method, a gravure printing method, a flexographic printing method, an offset printing method, or a microcontact printing method, and a Langmuir-Blodgett (LB) method.

### (Application and Printing)

In the present invention, a coating method or a printing method is preferably used as the method of applying the organic semiconductor composition onto the substrate. Particularly, it is preferable that the step of forming the organic semiconductor film includes a step of applying or printing the organic semiconductor composition on the substrate and then drying the organic semiconductor composition to form the organic semiconductor film.

Among the coating or printing methods, it is more preferable to use a drop casting method, a casting method, a spin coating method, an ink jet method, a gravure printing method, a flexographic printing method, an offset printing method, or a microcontact printing method.

In the present invention, it is particularly preferable to use a coating method.

Among the coating methods, a particularly preferable drop casting method will be described.

In the method of producing an organic semiconductor film using a drop casting method, it is preferable that the organic semiconductor composition is used as a coating solution, the coating solution is added dropwise onto a portion in the surface of a substrate A, the dropwise-added coating solution is slowly dried such that crystals of the compound represented by Formula 1 are precipitated, and thus a semiconductor active layer is formed.

Examples of the substrate Aused in the method of producing an organic semiconductor film using a drop casting method include a substrate used as a substrate of an organic transistor, and a substrate having an insulator layer formed on the substrate of an organic transistor is preferable.

### (Drying)

In the method of producing an organic semiconductor film using a drop casting method, it is preferable to slowly dry the dropwise-added coating solution such that the crystals of the compound represented by Formula 1 are precipitated to form a semiconductor active layer.

From the viewpoint of film quality, it is preferable that the heated substrate A is naturally dried and then dried under reduced pressure.

The temperature of the substrate A at the time of natural drying is preferably 20°C to 140°C, and more preferably 20°C to 120°C.

The natural drying time is preferably 5 minutes to 20 hours, more preferably 10 minutes to 10 hours.

In the method of producing an organic semiconductor film, it is preferable to precipitate the crystals of the compound represented by Formula 1. Whether or not the crystals are precipitated can be confirmed by observation with a polarization microscope. Examples

The features of the present invention will be described more specifically below with reference to Examples and Comparative Examples. Materials, amounts used, ratios, treatment contents, treatment procedures, and the like shown in the following examples can be appropriately changed without departing from the scope of the present invention. Therefore, the scope of the present invention should not be limitedly interpreted by the following specific examples.

In the compositions of Examples and Comparative Examples, the following compounds 1 to 8 satisfying Formula 1 and compounds 9 to 12 not satisfying Formula 1 were used. The "No." of each compound in Tables 1 to 3 is also shown next to the number assigned in Arabic numerals to each compound.

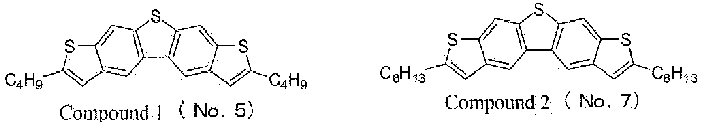

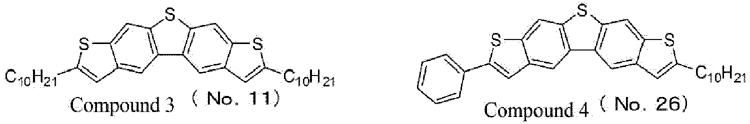

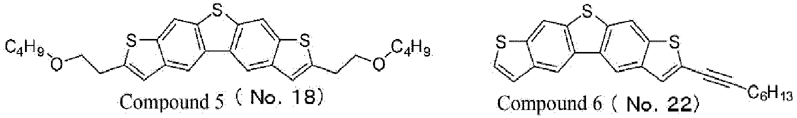

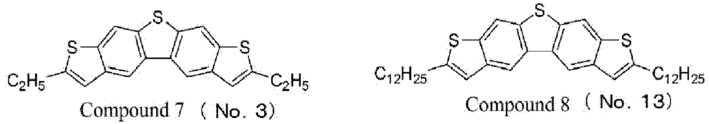

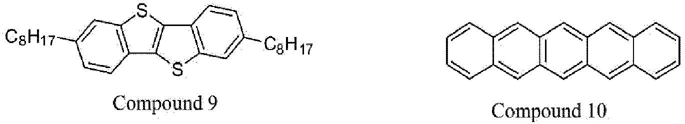

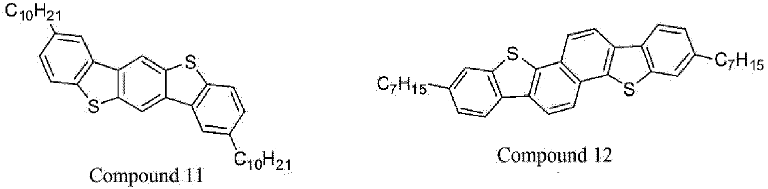

### [Examples 1 to 3 and Comparative Examples 1 to 9]

A compound 1 was used to prepare the composition of each of Examples and Comparative Examples. The compound 1 corresponds to the compound 4 in JP2015-195361A.

### <Synthesis of Compound 1 and Preparation of Composition of Comparative Example 1>

The compound 1 was synthesized and purified by recrystallization according to the method described in [0092] to [0095], particularly the purification method described in [0094] in JP2015-195361Ato prepare the composition of Comparative Example 1.

Specifically, after completion of the synthesis reaction, the composition was cooled to room temperature, 50 ml of methanol was added thereto, and the precipitated solid was separated by filtration. The solid was dissolved in heated O-dichlorobenzene, allowed to pass through celite and silica gel in a heated state, and eluted with heated O-dichlorobenzene. The obtained solution was concentrated with an evaporator and then recrystallized from the heated O-dichlorobenzene to obtain a composition of Comparative Example 1 as a white solid.

Using the composition of Comparative Example 1 obtained by purification by recrystallization described in [0094] of JP2015-195361A, the composition of Comparative Example 1 was further purified by the method described below. Thus, compositions of Examples 1 to 3 and Comparative Examples 2 to 10 were prepared.

### <Preparation of Composition of Example 1 by Sublimation Purification at Temperature of 150°C or Higher>

The composition (14 g) of Comparative Example 1 was set in a boat, and the temperature was raised from 150°C to 190°C at 10° C/10 minutes while flowing an argon gas under the condition of 2.0 mPa, and then sublimation purification was performed under the conditions at 200°C for 80 minutes and at 215°C to 220°C for 10 hours to obtain a composition of Example 1.

In addition, in Tables 4 and 5 below, the temperature in a state which the temperature is not raised is described as the sublimation purification temperature.

### <Preparation of Composition of Example 2 by Soxhlet Extraction>

The composition of Comparative Example 1 (1 g) was set in a Soxhlet extractor, extracted with 50 ml of toluene, concentrated, and then dried under reduced pressure to obtain a composition of Example 2. This purification method was designated as "Soxhlet Extraction A".

### <Preparation of Composition of Example 3 by Soxhlet Extraction>

The composition (1 g) of Comparative Example 1 was set in a Soxhlet extractor, extracted with a mixed solvent of 50 ml of toluene and 10 ml of methanol, concentrated, and then dried under reduced pressure to obtain a composition of Example 3. This purification method was designated as "Soxhlet extraction B".

### <Preparation of Composition of Comparative Example 2 by One Time of Water Dispersion>

The composition (1 g) of Comparative Example 1 and 50 ml of distilled water were placed in a 100 ml flask, and the mixture was stirred for 1 hour, and filtered. Thereafter, the filter cake was dried under reduced pressure to obtain a composition of Comparative Example 2.

### <Preparation of Composition of Comparative Example 3 by Two Times of Water Dispersion>

In a 100 ml flask, the composition of Comparative Example 2 (0.6 g), which had been dispersed once in water, and 30 ml of distilled water were placed, and the mixture was stirred for 1 hour and filtered. Thereafter, the filter cake was dried under reduced pressure to obtain a composition of Comparative Example 3.

### <Preparation of Composition of Comparative Example 4 by Three Times of Water Dispersion>

In a 100 ml flask, the composition of Comparative Example 3 (0.3 g), which had been dispersed twice in water, and 25 ml of distilled water were placed, and the mixture was stirred for 1 hour and filtered. Thereafter, the filter cake was dried under reduced pressure to obtain a composition of Comparative Example 4.

### <Preparation of Composition of Comparative Example 5 by One Time of Recrystallization>

In a 100 ml flask, the composition (1 g) of Comparative Example 1 and 20 ml of ortho-dichlorobenzene were placed, and the mixture was dissolved by heating, cooled with water, and filtered. Thereafter, the filter cake was dried under reduced pressure to obtain a composition of Comparative Example 5.

### <Preparation of Composition of Comparative Example 6 by Two Times of Recrystallization>

In a 100 ml flask, the composition (0.6 g) of Comparative Example 5, which had been recrystallized once, and 12 ml of ortho-dichlorobenzene were placed, and the mixture was dissolved by heating, cooled with water, and filtered. Thereafter, the filter cake was dried under reduced pressure to obtain a composition of Comparative Example 6.

### <Preparation of Composition of Comparative Example 7 by Three Times of Recrystallization>

In a 100 ml flask, the composition of Comparative Example 6 (0.3 g), which had been recrystallized twice, and 6 ml of ortho-dichlorobenzene were placed, dissolved by heating, cooled with water, and filtered. Thereafter, the filter cake was dried under reduced pressure to obtain a composition of Comparative Example 7.

### <Preparation of Composition of Comparative Example 8 by One Time of Silica Gel Chromatography>

The composition (2 g) of Comparative Example 1 was dissolved in chloroform and subjected to silica gel chromatography. The obtained fraction was concentrated under reduced pressure and dried under reduced pressure to obtain a composition of Comparative Example 8.

### <Preparation of Composition of Comparative Example 9 by Two Times of Silica Gel Chromatography>

The composition (1 g) of Comparative Example 8, which had been subjected to silica gel chromatography once, was dissolved in chloroform and subjected to silica gel chromatography. The obtained fraction was concentrated under reduced pressure and dried under reduced pressure to obtain a composition of Comparative Example 9.

### [Examples 4 to 6 and Comparative Examples 10 to 13]

A compound 2 was used to prepare compositions of each of Examples and Comparative Examples. The compound 2 corresponds to the compound 1a in Tetrahedron 66 (2010) 8778-8784.

### <Synthesis of Compound 2 and Preparation of Composition of Comparative Example 10>

The synthesis of the compound 2 and purification by column chromatography were performed by the method described in Tetrahedron 66 (2010) 8778-8784, particularly, the purification method described in 3.2.11. to prepare a composition of Comparative Example 10.

### <Preparation of Compositions of Examples 4 to 6 and Comparative Examples 11 to 13>

The composition of Comparative Example 2 obtained by purification by the column chromatography described in Tetrahedron 66 (2010) 8778-8784 was used, and the composition of Comparative Example 2 was purified in the same method as in Example 1 except for heating conditions to prepare a composition of Example 4. In Example 4, the heating conditions after temperature rising in the decompression purification were changed to a temperature of 210°C to 240°C and a heating time of 10 hours.

In addition, the composition of Comparative Example 2 was further purified in the same method as in Examples 2 and 3 and Comparative Examples 2 to 4 to prepare compositions of Examples 5 and 6 and Comparative Examples 11 to 13.

### [Example 7 and Comparative Example 14]

A compound 3 was used to prepare compositions of each of Example and Comparative Example. The compound 3 corresponds to the compound 1 in JP2015-195361A.

### <Synthesis of Compound 3 and Preparation of Composition of Comparative Example 14>

The compound 3 was synthesized and purified by recrystallization according to the method described in [0092] to [0094], particularly the purification method described in [0094] in JP2015-195361A to prepare a composition of Comparative Example 14. Specifically, recrystallization from O-dichlorobenzene was performed in the same manner as in Comparative Example 1 to obtain a composition of Comparative Example 14 as a white solid.

### <Preparation of Composition of Example 7>

The composition of Comparative Example 14 obtained by purification by recrystallization described in [0094] of JP2015-195361A was used, and the composition of Comparative Example 14 was further purified in the same method as in Example 2 to prepare a composition of Example 7.

### [Example 8 and Comparative Example 15]

A compound 4 was used to prepare compositions of each of Example and Comparative Example.

### <Synthesis of Compound 4 and Preparation of Composition of Comparative Example 15>

The compound 4 was synthesized by the following scheme with reference to the method described in JP2015-195361A.

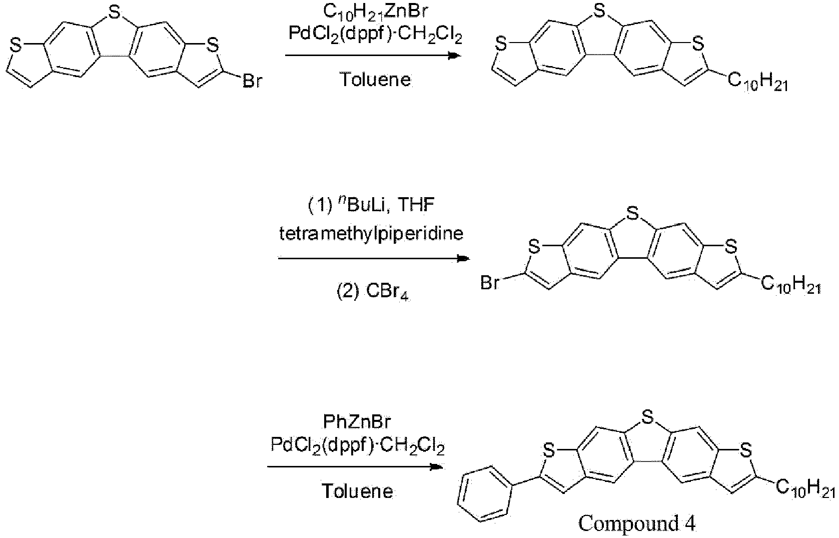

The purification by recrystallization after the synthesis reaction was performed according to the purification method described in [0094] of JP2015-195361A to prepare a composition of Comparative Example 15. Specifically, the solution was recrystallized from O-dichlorobenzene in the same manner as in Comparative Example 1 to obtain a composition of Comparative Example 15 as a white solid.

### <Preparation of Composition of Example 8>

The composition of Comparative Example 15 obtained by purification by recrystallization as described in [0094] of JP2015-195361A was used and the composition of Comparative Example 15 was further purified in the same method as in Example 1 except for the heating conditions. Thus, a composition of Example 8 was prepared. In Example 8, the heating conditions after temperature rising in the decompression purification were changed to a temperature of 230°C to 260°C and a heating time of 10 hours.

### [Example 9 and Comparative Example 16]

A compound 5 was used to prepare compositions of each of Example and Comparative Example. The compound 1 corresponds to the compound 18 in JP2015-195361A.

### <Synthesis of Compound 5 and Preparation of Composition of Comparative Example 16>

The synthesis of the compound 5 and purification by column chromatography were performed according to the method described in [0099] of JP2015-195361A to prepare a composition of Comparative Example 16.

Specifically, after the completion of the synthesis reaction, the reaction solution was cooled to room temperature and separated using chloroform and pure water. An organic layer was concentrated through distillation under reduced pressure and then purified by column chromatography (sequential development using silica gel, hexane:ethyl acetate=3:1, hexane:ethyl acetate=2:1, and hexane:ethyl acetate=1:1), thereby obtaining a composition of Comparative Example 16 as a white solid.

### <Preparation of Composition of Example 9>

The composition of Comparative Example 16 obtained by purification by the column chromatography described in [0099] of JP2015-195361A was used, and the composition of Comparative Example 16 was further purified by the same method as in Example 4 to prepare a composition of Example 9.

### [Example 10 and Comparative Example 17]

A compound 6 was used to prepare compositions of each Example and Comparative Example.

### <Synthesis of Compound 6 and Preparation of Composition of Comparative Example 17>

The compound 6 was synthesized by the following scheme with reference to the method described in JP2015-195361A.

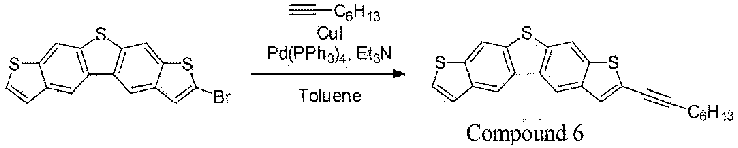

The purification by recrystallization after the synthesis reaction was performed according to the purification method described in [0094] of JP2015-195361A to prepare a composition of Comparative Example 17. Specifically, the solution was recrystallized from O-dichlorobenzene in the same manner as in Comparative Example 1 to obtain a composition of Comparative Example 17 as a white solid.

### <Preparation of Composition of Example 10>

The composition of Comparative Example 17 obtained by purification by recrystallization as described in [0094] of JP2015-195361A was used and the composition of Comparative Example 17 was further purified in the same method as in Example 1 except for the heating conditions. Thus, a composition of Example 10 was prepared. In Example 10, the heating conditions after temperature rising in the decompression purification were changed to a temperature of 190°C to 220°C and a heating time of 10 hours.

### [Example 11 and Comparative Example 18]

A compound 7 was used to prepare compositions of each of Example and Comparative Example.

### <Synthesis of Compound 7 and Preparation of Composition of Comparative Example 18>

The compound 7 was synthesized with reference to the method described in [0092] to [0095] of JP2015-195361A. Then, the compound 7 was purified by recrystallization according to the purification method described in [0094] of JP2015-195361A to prepare a composition of Comparative Example 18. Specifically, the solution was recrystallized from O-dichlorobenzene in the same manner as in Comparative Example 1 to obtain a composition of Comparative Example 18 as a white solid.

### <Preparation of Composition of Example 11>

The composition of Comparative Example 18 obtained by purification by recrystallization as described in [0094] of JP2015-195361A was used and the composition of Comparative Example 18 was further purified in the same method as in Example 1 except for the heating conditions. Thus, a composition of Example 11 was prepared. In Example 11, the heating conditions after temperature rising in the decompression purification were changed to a temperature of 190°C to 220°C and a heating time of 10 hours.

### [Example 12 and Comparative Example 19]

A compound 8 was used to prepare compositions of each Example and Comparative Example. The compound 8 corresponds to the compound 1b in Tetrahedron 66 (2010) 8778-8784.

### <Synthesis of Compound 8 and Preparation of Composition of Comparative Example 19>

The synthesis of the compound 8 and purification by column chromatography were performed by the method described in Tetrahedron 66 (2010) 8778-8784, particularly, the purification method described in 3.2.11. cited in 3.2.13. to prepare a composition of Comparative Example 10.

### <Preparation of Composition of Example 12>

A composition of Comparative Example 19 obtained by purification by column chromatography described in Tetrahedron 66 (2010) 8778-8784 was used and the composition of Comparative Example 19 was further purified in the same method as in Example 2 to prepare a composition of Example 12.

### [Comparative Example 101 and Comparative Example 102]

A compound 9 was used to prepare compositions of each Comparative Example. The compound 9 corresponds to the compound 1 in WO2015/133402A1.

As the composition containing the compound 9, a trade name C8-BTBT (trade number 747092) manufactured by Sigma-Aldrich Co. was purchased to obtain a composition of Comparative Example 101.

The composition of Comparative Example 102 was prepared by further purifying the composition of Comparative Example 101 in the same manner as in Example 1 except for heating conditions. In Comparative Example 102, the heating conditions after temperature rising in the decompression purification were changed to a temperature of 180°C to 240°C and a heating time of 10 hours.

### [Comparative Example 103 and Comparative Example 104]

A compound 10 was used to prepare compositions of each Comparative Example. The compound 10 corresponds to pentacene before purification described in [0073] of JP2003-347624A.

As the composition containing the compound 9, pentacene (trade number P1802) manufactured by Sigma-Aldrich Co. was purchased and used as a composition of Comparative Example 103.

The composition of Comparative Example 103 was further purified by the same method in Comparative Example 102 to prepare a composition of Comparative Example 104.

### [Comparative Example 105 and Comparative Example 106]

A compound 11 was used to prepare composition of each Comparative Example. The compound 11 corresponds to the compound 2 in WO2015/133402A1.

### <Synthesis of Compound 11 and Preparation of Composition of Comparative Example 105>

The synthesis of the compound 11 and purification by silica gel column chromatography were performed according to the method described in [0097] of WO2015/133402A1 to prepare a composition of Comparative Example 105. Regarding the purification method, specifically, the reaction solution was washed with water, concentrated, and then purified by silica gel column chromatography (methylene chloride-hexane mixed solvent) to obtain a solid. A part of the solid was added to Pd/C and a toluene-acetic acid mixed solvent, and the mixture was stirred under a hydrogen atmosphere for 14 hours. The reaction solution was filtered, concentrated, and purified by silica gel column chromatography (methylene chloride-hexane mixed solvent).

### <Preparation of Composition of Comparative Example 106>

The composition of Comparative Example 105 was further purified in the same method as in Example 2 to prepare a composition of Comparative Example 106.

### [Comparative Example 107 and Comparative Example 108]

A compound 12 was used to prepare compositions of each Example and Comparative Example. The compound 12 corresponds to the compound 4 in WO2015/133402A1.

### <Synthesis of Compound 12 and Preparation of Composition of Comparative Example 107>

The synthesis of the compound 12 and purification by silica gel column chromatography were performed according to the method described in [0099] and [0097] of WO2015/133402A1 to prepare a composition of Comparative Example 107. Specifically, the purification by silica gel column chromatography was performed in the same manner as in Comparative Example 105 to obtain a composition of Comparative Example 107.

### <Preparation of Composition of Comparative Example 108>

The composition of Comparative Example 107 was further purified in the same method as in Example 2 to prepare a composition of Comparative Example 108.

### [Preparation of Organic Semiconductor Element]

Through high performance liquid chromatography, it was confirmed that the materials used for preparation of organic semiconductor elements had a purity (area ratio for absorption intensity at 254 nm) of 99.0% or higher.

### <Preparation of Organic Semiconductor Composition>

Any one of the compositions of each of Examples and Comparative Examples prepared into a 0.1% by mass solution by being dissolved in anisole as a solvent and then the solution was heated to 50°C to prepare the organic semiconductor compositions of each of Examples and Comparative Examples.

### <Film Formation of Organic Semiconductor Film>

The organic semiconductor composition was applied onto a substrate by a drop casting method, dried, and formed into a film to form an organic semiconductor film.

A 25 mm × 25 mm substrate prepared by forming a thermally oxidized SiO₂ film having a thickness of 200 nm on the surface of an n-type silicon substrate (thickness: 0.4 mm) was used as the substrate A. The surface of the thermally oxidized film of the substrate A was cleaned with UV-ozone and then treated with β-phenethyltrimethoxysilane.

On the surface of the substrate A treated with β-phenethyltrimethoxysilane, the organic semiconductor composition of each of Examples and Comparative Examples was applied by drop casting, and then the substrate was dried for 10 minutes at 100°C on a hot plate to form an organic semiconductor film.

The obtained organic semiconductor film was used as a semiconductor active layer and further covered with a mask. Then, F4-TCNQ with a thickness of 1 nm as a charge injection acceptor and a gold electrode with a thickness of 40 nm were respectively vapor-deposited thereon, thereby obtaining a bottom gate/top contact-type organic transistor element for measuring FET characteristics. The obtained organic transistor element was used as an organic semiconductor element in each of Examples and Comparative Examples.

### [Evaluation]

### <Content of Element>

The compositions of Examples and Comparative Examples were subjected to ashing and acid dissolution, and the contents of sodium element, potassium element, silicon element, and aluminum element were quantified in ppm respectively by using ICP-MS HP 7700 manufactured by Agilent Technologies, Inc.

The total contents of sodium element, potassium element, silicon element, and aluminum element and the content of the silicon element were calculated and were shown in Tables 4 and 5 below.

The specific elements of the organic semiconductor film in the organic semiconductor element can be quantified in the same manner.

### <Heat Resistance of Carrier Mobility>

### (a) Carrier Mobility Before Heating

The FET characteristics of the organic semiconductor element were evaluated under normal pressure and atmospheric pressure using a semiconductor parameter analyzer (4156C manufactured by Agilent Technologies, Inc.) connected to a semi-automatic prober (AX-2000 manufactured by Vector Semiconductor Co., Ltd.).

Between the source electrode and the drain electrode of each organic film transistor element (FET element) of Examples and Comparative Examples, a voltage of -80 V was applied, and the gate voltage was changed within a range of 20 V to -100 V. In this manner, a carrier mobility µ was calculated using an equation showing a drain current I_{d}: I_{d} = (w/2L)µCᵢ (V_{g} - Vtₕ)². In the equation, L represents the gate length, w represents the gate width, Ci represents the capacity per unit area of the insulator layer, V_{g} represents the gate voltage, and Vtₕ represents the threshold voltage.

### (b) Carrier Mobility After Heating

After heating the organic semiconductor elements of each of Examples and Comparative Examples under the conditions of 120°C for 10 minutes in the atmosphere, the carrier mobility µ was measured by the same method as in the measurement of the carrier mobility before heating. The heating conditions are assumed as the heating conditions of a photoresist used for patterning an organic semiconductor film, for example, product name MICROPOSIT S1828 PHOTO RESIST manufactured by Shipley Company Inc.

### (c) Evaluation of Heat Resistance of Carrier Mobility

A maintenance rate of the carrier mobility after heating was calculated from the following equation.

Maintenance rate (%) of carrier mobility after heating = 100% × carrier mobility after heating/carrier mobility before heating

The maintenance rate of the carrier mobility after heating was evaluated according to the following evaluation standards to evaluate the heat resistance of carrier mobility. Practically, AA, A, or B is preferable, AA or A is more preferable, and AA is particularly preferable. The obtained results are shown in Tables 4 and 5 below.

### -Evaluation Standards-

AA: The maintenance rate of the carrier mobility was 95% or more.
A: The maintenance rate of the carrier mobility was 80% or more and less than 95%.
B: The maintenance rate of the carrier mobility was 65% or more and less than 80%.
C: The maintenance rate of the carrier mobility was 50% or more and less than 65%.
D: The maintenance rate of the carrier mobility was 35% or more and less than 50%.
E: The maintenance rate of the carrier mobility was 25% or more and less than 35%.
F: The maintenance rate of the carrier mobility was 15% or more and less than 25%.
G: The maintenance rate of the carrier mobility was less than 15%.

**[Table 4]**

| | Composition | | | | | Heat resistance of carrier mobility |
|---|---|---|---|---|---|---|
| | Compound | Purification method | Vacuum purification temperature (°C) | Total content of sodium element, potassium element, silicon element, and aluminum element (ppm) | Content of silicon element (ppm) | |
| Comparative Example 1 | 1 | Recrystallization described in [0094] of JP2015-195361A | - | 97 | 49 | E |
| Example 1 | 1 | After purification by method of Comparative Example 1, sublimation purification | 200 to 220 | 1 | <1 | AA |
| Example 2 | 1 | After purification by method of Comparative Example 1, Soxhlet extraction A | - | 12 | 2 | A |
| Example 3 | 1 | After purification by method of Comparative Example 1, Soxhlet extraction B | - | 48 | 27 | B |
| Comparative Example 2 | 1 | After purification by method of Comparative Example 1, one time of water dispersion | - | 62 | 42 | E |
| Comparative Example 3 | 1 | After purification by method of Comparative Example 1, two times of water dispersion | - | 55 | 32 | D |
| Comparative Example 4 | 1 | After purification by method of Comparative Example 1, three times of water dispersion | - | 54 | 31 | D |
| Comparative Example 5 | 1 | After purification by method of Comparative Example 1, one time of recrystallization | - | 75 | 40 | D |
| Comparative Example 6 | 1 | After purification by method of Comparative Example 1, two times of recrystallization | - | 64 | 37 | D |
| Comparative Example 7 | 1 | After purification by method of Comparative Example 1, three times of recrystallization | - | 63 | 35 | D |
| Comparative Example 8 | 1 | After purification by method of Comparative Example 1, one time of silica gel chromatography | - | 92 | 68 | D |
| Comparative Example 9 | 1 | After purification by method of Comparative Example 1, two times of silica gel chromatography | - | 90 | 72 | D |
| Comparative Example 10 | 2 | Column chromatography described in 3.2.11. of Tetrahedron 66 (2010) 8778-8784 | - | 122 | 75 | E |
| Example 4 | 2 | After purification by method of Comparative Example 10, sublimation purification | 210 to 240 | 1 | <1 | AA |
| Example 5 | 2 | After purification by method of Comparative Example 10, Soxhlet extraction A | - | 13 | 1 | A |
| Example 6 | 2 | After purification by method of Comparative Example 10, Soxhlet extraction B | - | 45 | 28 | B |
| Comparative Example 11 | 2 | After purification by method of Comparative Example 10, one time of water dispersion | - | 70 | 52 | D |
| Comparative Example 12 | 2 | After purification by method of Comparative Example 10, two times of water dispersion | - | 62 | 49 | D |
| Comparative Example 13 | 2 | After purification by method of Comparative Example 10, three times of water dispersion | - | 61 | 45 | D |

**[Table 5]**

| | Composition | | | | | Heat instance of carrier mobility |
|---|---|---|---|---|---|---|
| | Compound | Purification method | Vacuum purification temperature (°C) | Total content of sodium element, potassium element, silicon element, and aluminum element (ppm) | Content of silicon element (ppm) | |
| Comparative Example 14 | 3 | Recrystallization described in [0094] of JP2015-195361A | - | 84 | 52 | E |
| Example 7 | 3 | After purification by method of Comparative Example 14, Soxhlet extraction A | - | 12 | 2 | A |
| Comparative Example 15 | 4 | Recrystallization described in [0094] of JP2015-195361A | - | 74 | 41 | E |
| Example 8 | 4 | After purification by method of Comparative Example 15, sublimation purification | 230 to 60 | 3 | <1 | AA |
| Comparative Example 16 | 5 | Silica gel chromatography described in [0099] of JP2015-195361A | - | 115 | 72 | E |
| Example 9 | 5 | After purification by method of Comparative Example 16, sublimation purification | 210 to 240 | 4 | <1 | A |
| Comparative Example 17 | 6 | Recrystallization described in [0094] of JP2015-195361A | - | 214 | 85 | E |
| Example 10 | 6 | After purification by method of Comparative Example 17, sublimation purification | 190 to 220 | 3 | <1 | A |
| Comparative Example 18 | 7 | Recrystallization described in [0094] of JP2015-195361A | - | 121 | 65 | E |
| Example 11 | 7 | After purification by method of Comparative Example 18, sublimation purification | 180 to 210 | 2 | <1 | AA |
| Comparative Example 19 | 8 | Column chromatography described in 3.2.11. of Tetrahedron 66 (2010) 8778-8784 | - | 75 | 48 | E |
| Example 12 | 8 | After purification by method of Comparative Example 19, Soxhlet extraction A | - | 4 | 2 | A |
| Comparative Example 101 | 9 | - | - | 67 | 38 | G |
| Comparative Example 102 | 9 | Sublimation purification | 180 to 240 | 2 | <1 | F |
| Comparative Example 103 | 10 | - | - | 55 | 32 | E |
| Comparative Example 104 | 10 | Sublimation purification | 180 to 240 | 3 | <1 | E |
| Comparative Example 105 | 11 | Silica gel column chromatography described in [0097] of WO2015/133402A1 | - | 85 | 42 | c |
| Comparative Example 106 | 11 | After purification by method of Comparative Example 105, Soxhlet extraction A | - | 15 | 3 | c |
| Comparative Example 107 | 12 | Silica gel column chromatography described in [0097] of WO2015/133402A1 | - | 92 | 58 | C |
| Comparative Example 108 | 12 | After purification by method of Comparative Example 107, Soxhlet extraction A | - | 16 | 2 | C |

From Tables 4 and 5 above, it was found that the organic semiconductor elements using the compositions according to the embodiment of the present invention had high heat resistance of carrier mobility.

On the other hand, it was found that the organic semiconductor elements using the compositions of Comparative Examples had low heat resistance of carrier mobility. Particularly, in the compositions of Comparative Examples 1 to 19, a total content of sodium element, potassium element, silicon element, and aluminum element exceeded the upper limit defined in the present invention, and the compositions had low heat resistance of carrier mobility.

The compositions of Comparative Examples 101, 103, 105, and 107 contained a compound not satisfying Formula 1, and the total content of sodium element, potassium element, silicon element, and aluminum element exceeded the upper limit defined by the present invention. The heat resistance of carrier mobility was low. The compositions of Comparative Examples 102, 104, 106, and 108 contained a compound not satisfying Formula 1, and the heat resistance of carrier mobility was low.

From the above, it was found that the heat resistance of carrier mobility could not be increased only by reducing the total content of sodium element, potassium element, silicon element, and aluminum element. It was also found that in a case where the total content of sodium element, potassium element, silicon element, and the aluminum element was reduced and the compound represented by Formula 1 was selected, the heat resistance of carrier mobility could be increased.

### Explanation of References

- 11:: substrate
- 12:: electrode
- 13:: insulator layer
- 14:: semiconductor active layer (organic semiconductor film)
- 15a, 15b:: electrode
- 31:: substrate
- 32:: electrode
- 33:: insulator layer
- 34a, 34b:: electrode
- 35:: semiconductor active layer (organic semiconductor film)

## Claims

1. An organic semiconductor element comprising:
an organic semiconductor film formed by forming a composition into a film,
wherein the composition contains a compound represented by Formula 1, and
a total content of sodium element, potassium element, silicon element, and aluminum element in the composition is 50 ppm or less, 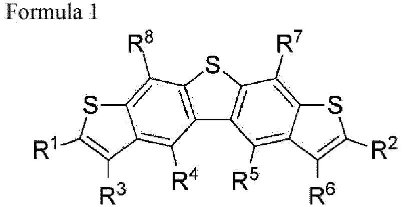
in Formula 1, R¹ to R⁸ each independently represent a hydrogen atom or a substituent.

2. The organic semiconductor element according to claim 1,
wherein the compound represented by Formula 1 is a compound represented by Formula 2, 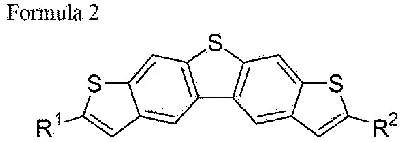 in Formula 2, R¹ and R² each independently represent an alkyl group which may have a substituent or an aromatic group which may have a substituent.

3. The organic semiconductor element according to claim 1 or 2,
wherein a content of the silicon element in the composition is 30 ppm or less.

4. The organic semiconductor element according to any one of claims 1 to 3,
wherein the organic semiconductor element is an organic transistor.

5. A composition comprising:
a compound represented by Formula 1,
wherein a total content of sodium element, potassium element, silicon element, and aluminum element in the composition is 50 ppm or less, 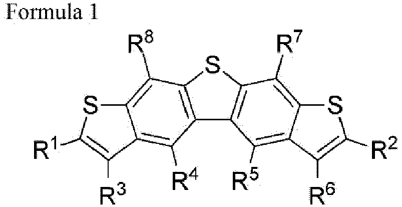
in Formula 1, R¹ to R⁸ each independently represent a hydrogen atom or a substituent.

6. The composition according to claim 5,
wherein a content of the silicon element in the composition is 30 ppm or less.

7. An organic semiconductor composition comprising:
the composition according to claim 5 or 6; and
a solvent.

8. An organic semiconductor film formed by forming the organic semiconductor composition according to claim 7 into a film.

9. A method of producing a composition comprising:
a step of subjecting a compound represented by Formula 1 to decompression purification at a temperature of 150°C or higher or Soxhlet extraction, 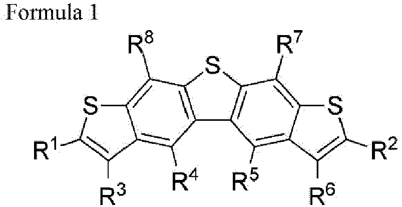
in Formula 1, R¹ to R⁸ each independently represent a hydrogen atom or a substituent.

10. A method of manufacturing an organic semiconductor element comprising:
a step of producing a composition by the method of producing a composition according to claim 9;
a step of producing an organic semiconductor composition by mixing the composition and a solvent; and
a step of forming an organic semiconductor film by forming the organic semiconductor composition into a film.

11. The method of producing an organic semiconductor element according to claim 10,
wherein the step of forming an organic semiconductor film includes a step of applying or printing the organic semiconductor composition onto a substrate, and then drying the organic semiconductor composition to form an organic semiconductor film.

12. A method of purifying a compound comprising:
subjecting a compound represented by Formula 1 to decompression purification at a temperature of 150°C or higher or Soxhlet extraction, 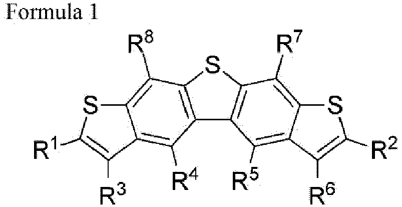
in Formula 1, R¹ to R⁸ each independently represent a hydrogen atom or a substituent.
